Europäisches Patentamt

(19) European Patent Office

Office européen des brevets

(11) Numéro de publication: **0 012 078**
**B1**

(12) **FASCICULE DE BREVET EUROPEEN**

(51) Int. Cl.³: **C 12 N 15/00**, C 12 P 21/02, **A 01 K 39/12**

(45) Date de publication du fascicule du brevet:
25.01.84

(21) Numéro de dépôt: 79400925.8

(22) Date de dépôt: 27.11.79

(54) Vecteur permettant l'insertion d'un gène procaryote ou eucaryote, et l'excrétion de la protéine exprimée.

(30) Priorité: 27.11.78 FR 7833498

(43) Date de publication de la demande:
11.06.80 Bulletin 80/12

(45) Mention de la délivrance du brevet:
25.01.84 Bulletin 84/4

(84) Etats contractants désignés:
**AT BE CH DE GB IT NL**

(56) Documents cités:
**C.R. ACAD. SC. Paris, Serie D, 288, 15 janvier 1979,
pages 275-277 C. MARCHAL et al. "Biologie
Moleculaire. Un Système de vecteurs destiné à
permettre l'excrétion d'une protéine déterminée par un
gène cloné**

(73) Titulaire: INSTITUT PASTEUR, 25-28, rue du Docteur
Roux, F-75724 Paris Cedex 15 (FR)

(72) Inventeur: Hofnung, Maurice, 144, rue Lecourbe,
F-75015 Paris (FR)
Inventeur: Perrin, David, 95, Boulevard Saint-Michel,
F-75005 Paris (FR)
Inventeur: Marchal, Christian, 39, Boulevard du
Montparnasse, F-75006 Paris (FR)

(74) Mandataire: Gutmann, Ernest et al, Cabinet
Plasseraud, 84, rue d'Amsterdam, F-75009 Paris (FR)

**Vecteur permettant l'insertion d'un gène procaryote ou eucaryote, et l'excrétion de la protéine exprimée**

L'invention est relative à de nouveaux vecteurs dans le génome desquels peuvent être insérés par fusion génétique, in vivo ou in vitro, un fragment d'ADN comportant un gène étranger susceptible de coder la production d'une protéine déterminée, procaryote ou eucaryote, ces vecteurs étant construits de telle façon qu'ils permettent non seulement l'expression de ce gène dans des cellules, notamment une bactérie, mais encore l'excrétion de la protéine produite.

Elle concerne encore, outre les vecteurs ainsi modifiés, les ADN ou ARN (acides désoxyribonucléiques ou ribonucléiques) correspondants.

On dira ci-après qu'une protéine est «excrétée» par une bactérie lorsqu'après synthèse elle se trouve localisée à l'extérieur de la membrane cellulaire ou est libérée (ou libérable) dans le milieu de culture.

On sait que le produit de traduction du gène lamB chez E. coli K-12 est une protéine de la membrane externe qui joue en particulier le rôle de récepteur bactérien pour le phage λ. L'étude de sa mise en place dans la membrane externe est susceptible d'apporter des informations sur le transfert des protéines à travers la membrane interne. Chez la souche sauvage, le gène lamB fait partie d'un des opérons de la région malB.

Plus particulièrement, Silhavy et Coll. [Proc. Natl. Acad. Sci. USA, vol. 74, n° 12, pp. 5415 (1977)] ont construit des souches qui leur ont permis de montrer qu'un ADN recombinant comportant un gène obtenu par fusion génétique in vivo du gène lamB (qui code le récepteur bactérien du phage λ) et d'un fragment de gène qui code une partie importante de la séquence COOH-terminal de la β-galactosidase (protéine cytoplasmique) pouvait être exprimé dans ces souches sous la forme d'une protéine hybride repérable à l'extérieur des membranes extérieures de leurs cellules. Ces ADN recombinants ne comportaient pas promoteur de l'opéron lactose.

L'invention a pour but de fournir des vecteurs mettant en œuvre les propriétés de gènes qui, tels le gène lamB, sont susceptibles de coder des protéines extracytoplasmiques susceptibles, soit d'être retrouvées dans la membrane extérieure de la cellule hôte, soit d'être directement excrétées dans le milieu de culture de cette cellule en vue de l'expression dans des bactéries de fragments de gène codant une protéine déterminée et de l'excrétion directe de cette protéine.

Le vecteur selon l'invention, qui est porteur du promoteur de l'opéron lactose et d'au moins un fragment du gène Z de ce même opéron, est caractérisé par le fait qu'au moins une séquence, plus particulièrement une séquence comportant la partie proximale d'un gène codant une protéine extracytoplasmique, notamment d'un récepteur bactérien pour un phage, ou au moins de son précurseur, se trouve insérée dans ce vecteur, plus particulièrement dans ce gène Z ou fragment de gène Z, ou immédiatement en aval de celui-ci, la longueur de cette séquence de gène extracytoplasmique étant suffisante pour permettre l'excrétion, sous le contrôle du promoteur de l'opéron lactose, de la protéine hybride susceptible d'être exprimée dans une souche bactérienne, dans laquelle ce vecteur aura été introduit au préalable.

Avantageusement, le gène codant une protéine extracytoplasmique est constitué par le gène lamB, la susdite séquence comprenant alors aussi, de préférence, la partie distale du gène K qui, dans les opérons maltose B, est associée au gène lamB.

Obtenir qu'une protéine issue d'un gène cloné soit excrétée est intéressant à plusieurs titres. On dispose ainsi d'un nouveau moyen d'étude des déterminants génétiques responsables de l'excrétion. Elle permet l'étude et le repérage plus aisé de la protéine exprimée. La protéine excrétée est, surtout s'il s'agit d'une protéine cytoplasmique, relativement protégée des protéases bactériennes. En outre, l'exposition à la surface peut faciliter la mise au point de vaccins contre des protéines virales et ce plus particulièrement dans le cas de protéines virales et dans le cas où la bactérie-hôte est inoffensive, de sorte que celle-ci peut être directement utilisée comme véhicule porteur des antigènes vaccinants que constituent ces protéines virales. Enfin, cette protéine – par exemple la somatostatine peut être séparée et retrouvée dans le milieu, de sorte que sa préparation en grande quantité est facilitée.

Il semble maintenant bien établi que les protéines excrétées sont synthétisées sous forme de précurseur. Le précurseur comporte un peptide N terminal d'environ 20 acides aminés ou peptide signal. Le peptide semble contenir des instructions suffisantes pour que la protéines soit excrétée; il est détaché au cours de l'excrétion. On peut envisager d'obtenir l'excrétion d'une protéine déterminée (x), en fusionnant par recombinaison à l'extrémité proximale de son gène de structure (X), un fragment de DNA (S) correspondant à au moins un peptide signal (s).

Un mode de mise en œuvre préféré de l'invention consiste à insérer, par fusion génétique, le gène X dans un vecteur comprenant la séquence S du gène lamB déterminant le récepteur bactérien pour le phage λ, plus particulièrement un fragment proximal du gène lamB et lui-même fusionné à un fragment du gène de structure de la β-galactosidase lacZ, y inclus le promoteur de l'opéron lactose. La protéine hybride obtenue possède l'extrémité N terminal du récepteur du phage λ. Il faut noter que la synthèse de la protéine hybride est alors soumise à la régulation du système lactose. Cette propriété permet en particulier en choisissant convenablement – et de façon en soi connue – la souche bactérienne qui héberge le phage, de déclencher l'excrétion par l'adjonction d'isopropylthiogalactoside (IPTG) au milieu du culture ou d'avoir une excrétion constitutive de la protéine hybride.

La protéine est susceptible d'être excrétée dès lors que la taille du fragment de récepteur est suffisante, correspondant notamment à un fragment protéique ayant un poids moléculaire atteignant, de préférence, de l'ordre de 2.000 (de préférence 2.500) dans le cas du récepteur du phage λ (la masse moléculaire du récepteur λ purifié étant de l'ordre de 55.000). Le fragment de récepteur du genre en question peut naturellement être plus important, notamment correspondre à un fragment de protéine ayant un poids moléculaire supérieur à 20.000, ou même à 30.000. Par exemple ce fragment de récepteur correspond à un protéine ayant une taille comprise entre 20.000 et environ 40.000, notamment entre environ 35.000 et environ 40.000.

Avantageusement, ce vecteur est constitué par un phage dont le ou les récepteurs bactériens correspondants sont distincts de celui auquel correspond la séquence de gène extracytoplasmique qui s'y trouve insérée. Par exemple, lorsque la séquence insérée correspond au gène *lam*B, on peut avoir recours à un vecteur dérivé du phage h°434.

Un tel type de vecteur permet:

a) l'insertion dans son génome, en aval du promoteur de l'opéron lactose du fragment d'ADN X, de préférence pourvu au préalable d'extrémités cohésives EcoRI;

b) l'introduction de l'ensemble du vecteur ainsi modifié dans des bactéries résistantes au phage λ grâce à la spécificité d'hôte h°434. Cette propriété permet d'examiner directement l'expression éventuelle du gène *lam*B inséré dans le vecteur. Il suffit de voir si les bactéries lysogènes pour le vecteur porteur de *lam*B ont recouvré la sensibilité au phage λ.

L'insertion susdite du fragment d'ADN X – correspondant par exemple au fragment de gène de la somatostatine décrit dans l'article instulé «Expression in Escherichia coli of a Chemically Synthesized Gene for the Hormone Somatosta tine» (Expression dans Escherichia coli d'un gène produit par synthèse chimique pour l'hormone somatostatine), publié par Keiichi Itakura et Coll. dans Science, vol. 198, pp. 1056–1063 du 9 décembre 1977 – intervient en aval de la séquence de gène correspondant au fragment de récepteur minimum requis pour l'excrétion, mais en amont de l'extrémité la plus éloignée du promoteur lactose, soit du gène Z ou fragment de gène Z, soit de la séquence de gène correspondant à la protéine extracytoplasmique, notamment *lam*B, dans le cas où cette séquence ne serait pas suivie d'un fragment distal de gène Z dans le sens de la traduction.

D'autres caractéristiques de l'invention apparaîtront encore au cours de la description qui suit de certains de ses modes de mise en œuvre préférés, notamment à l'aide des dessins dans lesquels:

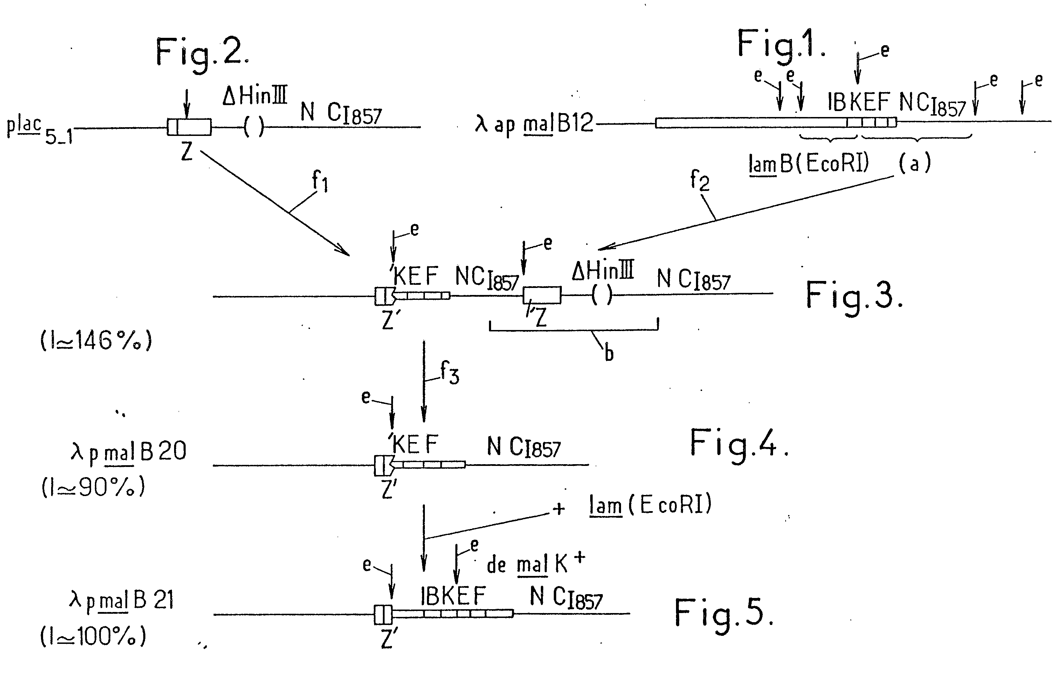

les fig. 1 à 12 sont respectivement représentatives (de façon schématique) des différentes formes de phages qui ont été mis en jeu; le promoteur de l'opéron maltose (entre les gènes K et E) n'étant pas représenté dans les vecteurs que portent les deux gènes en question.

Dans ces figures, les symboles:

K, E, F désignent les gènes K, E, F des opérons maltose B;

IB représente le gène *lam*B;

L est le promoteur de l'opéron lactose;

Z, Z∕, \ Z, Z∕∕ et \\Z désignent le gène Z ou des fragments du gène Z de l'opéron lactose, la position des signes ∕, \, ∕∕, \\étant indicative des coupures (fragments respectivement proximaux ou distaux, dans le sens de traduction des vecteurs considérés (de gauche à droite dans les fig. 1 à 5 et de droite à gauche dans toutes les autres);

X correspond au gène inséré et dont l'expression est recherchée;

Y et A correspondent respectivement aux gènes de la perméase et de l'acétylase ou des fragments de ceux-ci;

b, x, z correspondent aux protéines codées par les gènes IB, X et Z respectivement, ces lettres pouvant également être affectées des mêmes signes∕, \, ∕∕, \\, en conformité avec l'annotation des gènes correspondants dans les figures considérées.

Les autres indications sont conventionnelles soit de mutations, soit de délétions introduites dans les vecteurs schématiquement représentés.

1) Fabrication d'un fragment *lam*B (EcoRI)

Il a été obtenu par hydrolyse EcoRI de l'ADN du phage λap*lam*B12 (déposé à la Collection Nationale de Culture de Micro-organismes de l'institut Pasteur (C.N.C.M.) sous le n° I–072) et séparation des fragments par ultracentrifugation sur gradient de saccharose. Le phage λap*lam*B12 est schématiquement représenté dans la fig. 1. Les flèches $^e$y désignent les sites de coupures par l'enzyme EcoRI. On y voit également les emplacements respectifs du fragment *lam*B (EcoRI) et d'un fragment (a), lequel sera mis en jeu pour la fabrication d'un phage permettant le clonage du fragment *lam*B (EcoRI). Il a en effet été constaté que le gène *lam*B était dans les opérons maltose B entourés de deux sites EcoRI proches, dont l'un dans le gène K.

2) Clonage du fragment *lam*B (EcoRI)

Par recombinaison in vivo du phage λap*lam*B12 et d'un phage λp*lac*5–1 (C.N.C.M. n° 1–074) schématiquement représentés dans la fig. 2, on obtient le phage λp*mal*B20 représenté à la fig. 4, qui forme des plages et porte *mal*E$^+$. Il résulte de la transformation du phage intermédiaire, instable, représenté à la fig. 3, tel qu'il résulte de la recombinaison in vivo de λ*lac*$_5$-, et λap*mal*B12, par délétion interne in vivo de, la partie *b*. Le phage λp*mal*B20 est repéré en ce qu'il forme des particules transductrices de *mal*K$^+$. Les flèches f$_1$, f$_2$ et f$_3$ font apparaître la nature des transformations opérées.

On insère le fragment *lam*B (EcoRI) dans le phage λp*mal*B20 par recombinaison in vitro (insertion du fragment *lam*B (EcoRI) dans le site EcoRI de λp*mal*B20) et l'on repère le phage λp*mal*B21 (fig. 5) permettant le clonage du frag-

ment *lam*B (EcoRI), en tant que particules transductrices de *mal*K+.

Les longueurs 1 (ordres de grandeurs exprimés par le signe ≈) des phages des fig. 3, 4 et 5 sont exprimées en % de la longueur du phage λ. Le signe indique les emplacements de fusion des gènes du genre en question.

3) Fabrication d'un vecteur dérivé d'un phage autre que le phage λ et contenant le gène *lam*B sous le contrôle du promoteur de l'opéron lactose

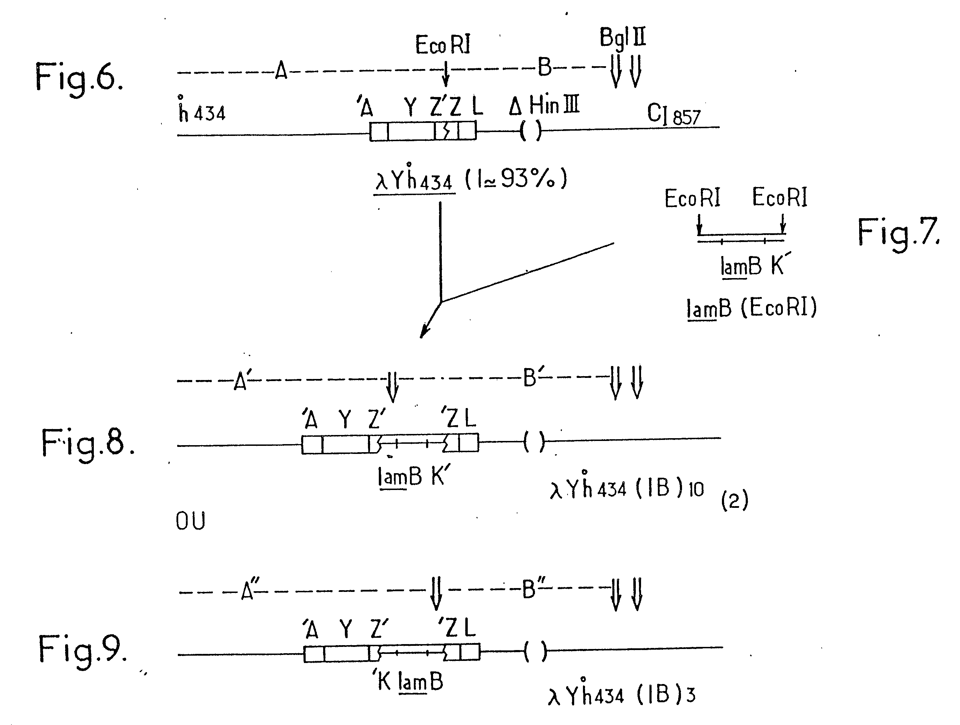

Le fragment *lam*B (EcoRI) a été inséré dans un vecteur porteur de l'opérateur et du promoteur (affecté de la mutation L8UV5) de l'opéron lactose: λYh°434 (fig. 6). L'insertion a été réalisée par hydrolyse complète du vecteur par EcoRI) et ligation en présence du fragment *lam*B (EcoRI) (fig. 7) par la ligase du phage T4. Par transfection d'une indicatrice *lac⁻*, des bactéries lysogènes pour des phages porteurs de l'insertion ont pu être isolées.

Le vecteur λYh°434, qui comporte les fonctions tardives donnant la spécificité d'hôte 434, a été obtenu par croisement d'un phage 'λ·h°434 (C.N.C.M. n° 1–070) dépourvu de site de coupure EcoRI (résultant lui-même d'une hybridation entre un phage λ et un phage 434), d'une part, et d'un phage λY (C.N.C.M. n° I–071), d'autre part, préalablement fabriqué et comportant dans son génome une partie de l'opéron lactose, notamment son promoteur L, un fragment de gène Z (Z, Z') comportant un site unique EcoRI et un fragment A' du gène de l'acétylase. Le phage λYh°434 est en outre pourvu de sites correspondant à l'enzyme de restriction BglII (représentés par des doubles flèches) qui permettent ensuite la vérification, par hydrolyse de ces sites, de l'orientation correcte du fragment *lam*B (EcoRI), lorsque celui-ci sera inséré dans ce vecteur.

a: La présence du fragment et l'expression éventuelle du gène *lam*B ont été examinées par recombinaison et complémentation dans des souches lysogènes pour les vecteurs ayant inséré le fragment *lam*B (EcoRI) préalablement construit.

a1) Des recombinants «maltose plus» ont été obtenus dans deux souches porteuses de mutations ponctuelles dans la partie distale du gène *mal*K. C'est la preuve de la présence de cette partie du gène sur le fragment porté par le vecteur.

a2) Quatre souches initialement résistantes au phage λ pour des raisons différentes lui deviennent sensibles. Les propriétés génétiques de ces souches permettent de conclure que cette sensibilité est alors:

due au gène *lam*B porté par le vecteur;

indépendante de l'expression du sytème maltosé; elle est observée en l'absence du produit du gène régulateur positif *mal*T;

insensible à la répression catabolique: ceci est conforme au fait qu'elle est sous la dépendance du promoteur L8UV5;

a3) Une souche *mal*+, mais incapable de croître sur dextrines à la suite d'une mutation, devient capable d'utiliser les dextrines mais uniquement en présence d'IPTG. Ceci indique que l'expression

du gène *lam*B du vecteur est inductible par l'IPTG.

b: La présence du fragment *lam*B (EcoRI) et son sens d'intégration dans le vecteur λYh°434 ont été mis en évidence sur gel d'agarose en utilisant des préparations de DNA de deux phages exprimant le gène *lam*B et d'un phage ne l'exprimant pas.

L'analyse des résultats permet de conclure que le phage en question a intégré le fragment *lam*B (EcoRI) dans le sens permettant l'expression de *lam*B à partir du promoteur de l'opéron *lac*, ce que ne peuvent pas les phages de recombinaison ayant intégré ce fragment dans le sens inverse (fig. 9).

4) Fabrication d'un vecteur λLBZ, dérivé d'un phage λ et contenant le gène *lam*B sous le contrôle du promoteur de lopéron lactose

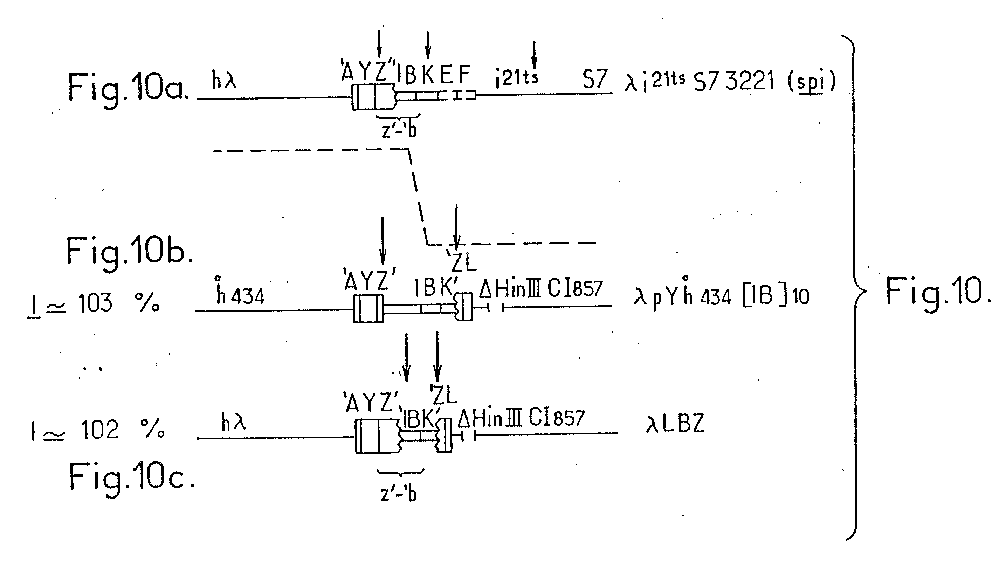

On fabrique d'abord le phage λi²¹ᵗˢSam7 3221 (*Spi*) (C.N.C.M. n° I–075), porteur de la fusion de gènes 42–1 entre *lam*B et *lac*Z, par recombinaison de l'ADN obtenu à partir de la souche pop 3221, décrite dans la publication de Silhavy et al déjà mentionnée, d'une part, et d'un phage λ, d'autre part (fig. 10a).

Par recombinaison in vivo entre le phage λi²¹ᵗˢSam7 3221 (*spi*) et le phage λpYh°434 (*1B*)10 (fig. 8 également reproduit à la fig. 10b), on obtient le phage λLBZ (fig. 10c), dans lequel le gène *lam*B est désormais sous contrôle du promoteur de l'opéron lactose. La ligne brisée de la fig. 10, entre les fig. 10a et 10b, fait apparaître les parties du phage λLBZ qui proviennent des phages des fig. 10a et 10b respectivement. Le phage λpYh°434(*1B*)10 porte la mutation *mal*K5. Le phage λLBZ aussi. L'expression de la fusion de gènes 42–1 se trouve donc sous contrôle du promoteur de l'opéron lactose.

Dans la fig. 10 se trouvent donc:

ε = fusion de gènes donnant une protéine hybride,

 = protéine hybride,

L = la présence de la mutation du promoteur L8UV5 de l'opéron lactose,

l = longueur approximative en pourcentage de celle du phage λ.

5) Fabrication d'un phage h°434 modifié comprenant à la fois un fragment de l'opéron lactose et le gène X

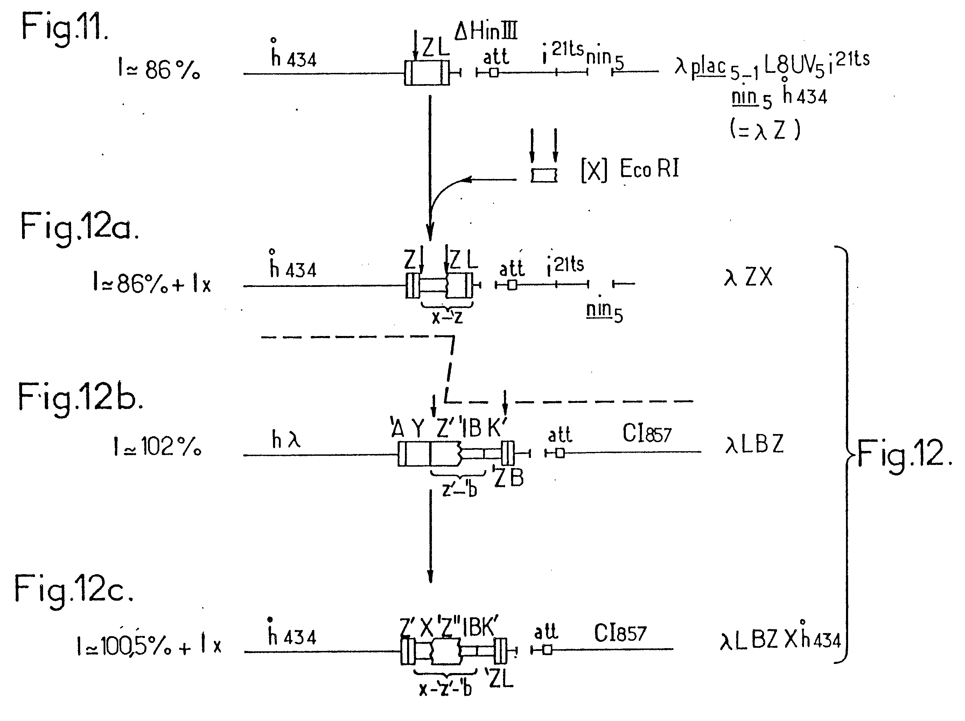

Le fragment EcoRI contenant le gène X – tel que celui de la somatostatine – est inséré dans le site EcoRI d'un vecteur λp*lac*5–1 L8UV5 i²¹ᵗˢ*nin*5 h°434 par recombinaison in vitro. Ce vecteur (fig. 11) possède la spécificité l'hôte h°434. L'insertion du fragment [X] EcoRI conduit à la formation d'une protéine hybride z'–x. Le vecteur λp*lac* 5–1 L8UV5 i²¹ᵗˢ *nin*5 h°434 est issu d'un croisement entre λap*mal*B21 h°434 (recombinant entre λp*mal*B21 et λ·h°434) et λp*lac*5–1 L8UV5 *nin*5 (C.N.C.M. n° I–073).

Le phage obtenu, aussi dénommé λZX, est représenté à la fig. 12a.

6) Fabrication du phage contenant à la fois le gène X et le gène *lam*B

Le phage contenant l'insertion du gène X (fig. 12a) est croisé avec le phage λLBZ (représenté tant à la fig. 10c qu'à la fig. 12b). On repère les phages produits par recombinaison in vivo au sein du gène lacZ. La ligne brisée de la fig. 12 met en évidence les parties constitutives des recombinants obtenus (fig. 12c). Ils sont porteurs d'une double fusion de gène qui conduit à la formation d'une protéine hybride b'–z'–x, qui peut être excrétée, lorsque ces phages sont exprimés dans une bactérie dépourvue de récepteurs pour le phage λ, dans laquelle le susdit phage a au préalable été introduit.

Lorsque x est la somatostatine humaine, elle peut ensuite être récupérée par traitement des bactéries porteuses, dans des conditions analogues à celles décrites dans l'article d'Itakura et Coll. susmentionné, pour ce qui est de l'isolement de la somatostatine humaine à partir de la protéine hybride que ces auteurs avaient obtenue.

En constituant le gène X par un fragment d'ADN d'adénovirus 5 porteur d'extrémités EcoRI* (E coRl étoile), on dispose d'un vecteur modifié dont l'expression est susceptible de se manifeste par l'obtention d'une protéine hybride extracytoplasmique comprenant une protéine à caractère antigénique correspondant à l'adénovirus 5.

Des modifications permettent au système de vecteur décrit d'accepter un fragment d'ADN de taille plus importante. Elles consistent par exemple à changer par recombinaison in vivo les fonctions précoces et tardives du λZ et du λLBZ pour obtenir finalement un dérivé λi²¹ᵗˢnin5 LBZXhλ dont l'ADN a une taille inférieure à celle de λLBZX de plus de 10%. En outre, il est possible d'introduire des délétions au sein du gène lacZ de façon à diminuer la taille du double hybride. Les délétions pourraient également entraîner des modifications de conformation du double hybride.

Un deuxième système de vecteurs peut être également élaboré à partir de phages comme le λLBZ. En effet, la séquence d'ADN correspondant au début du gène lamB (et donc au peptide signal) est en cours de détermination au laboratoire. Connaissant cette séquence, on peut penser qu'il sera possible d'insérer directement un gène X en bonne position dans le gène lamB de façon à ce que les protéines hybrides résultant de cette fusion soient excrétées.

Les phages modifiés ainsi obtenus ou une partie de ces phages comprenant notamment le promoteur de l'opéron lactose, le gène Z ou fragment de gène Z et le fragment d'ADN comportant le gène lamB ou séquence de gène lamB (ou tout autre gène ou séquence de gène codant la fabrication d'une protéine extracytoplasmique) pourraient être insérés dans un autre système de vecteur, par exemple un plasmide ou un chromosome bactérien.

Ainsi pourrait-on par exemple insérer le phage vecteur ou la partie de phage vecteur susmentionnée, par exemple dans un plasmide tel que le plasmide pBR322. De tels vecteurs modifiés seraient appropriés à l'expression de protéines de grand poids moléculaire, telles que par exemple l'ovalbumine de poulet.

Comme il va de soi et comme il résulte d'ailleurs déjà de ce qui précède, l'invention ne se limite nullement à ceux de ses modes d'application et de réalisation qui ont été plus spécialement envisagés; elle en embrasse au contraire toutes les variantes, notamment celles où l'on aurait recours à des coupures réalisées au niveau d'autres sites, par exemple ceux correspondant à d'autres enzymes de restriction, par exemple l'enzyme Sst.

**Revendications**

1. Vecteur porteur du promoteur de l'opéron lactose et d'au moins un fragment du gène Z de ce même opéron, caractérisé par l'insertion dans ce vecteur d'au moins une séquence comportant la partie proximale d'un gène codant au moins une partie d'une protéine extracytoplasmique normalement localisée dans la membrane externe de la cellule-hôte d'origine ou susceptible d'être libérée dans un milieu de culture de ces dernières, la longueur de cette séquence de gène extracytoplasmique étant suffisante pour permettre l'excrétion, sous le contrôle du promoteur de l'opéron lactose, d'une protéine hybride contenant ladite partie de protéine cytoplasmique et susceptible d'être exprimée dans une culture de cellules, notamment d'une souche bactérienne, dans lesquelles ce vecteur aura été introduit au préalable.

2. Vecteur selon la revendication 1, caractérisé par le fait que ladite séquence d'insertion est constituée par celle qui est apte à coder au moins la partie proximale d'un récepteur bactérien, tel que le récepteur pour le phage λ.

3. Vecteur selon la revendication 1 ou la revendication 2, caractérisé en ce que ladite séquence d'insertion est insérée dans le gène Z ou fragment de gène Z, ou immédiatement en aval de celui-ci.

4. Vecteur selon l'une quelconque des revendications 1 à 3, caractérisé par le fait que la susdite séquence comporte le gène lamB et aussi la partie distale
distale du gène K qui, dans les opérons maltose B, est associée au gène lamB.

5. Vecteur selon la revendication 4, caractérisé par le fait qu'il contient la séquence S du gène lamB déterminant le récepteur bactérien pour le phage λ, plus particulièrement un fragment proximal du gène lamB et lui-même fusionné à un fragment du gène de structure de la β-calactosidase lacZ.

6. Vecteur selon la revendication 4 ou 5, caractérisé par le fait que la taille du susdit fragment proximal de gène lamB susdit correspond à un fragment protéique ayant un poids moléculaire atteignant au moins 2.000, de préférence 2.500.

7. Vecteur selon la revendication 6, caractérisé par le fait que la taille du susdit fragment proximal de gène lamB susdit correspond à un fragment protéique ayant un poids moléculaire atteignant au moins 20.000, de préférence au moins 30.000.

8. Vecteur selon la revendication 7, caractérisé par le fait que la taille du susdit fragment proximal de gène, lamB susdit correspond à un fragment protéique ayant un poids moléculaire compris entre environ 20.000 et environ 40.000, notamment entre environ 35.000 et environ 40.000.

9. Vecteur selon l'une quelconque des revendications 1 à 8, caractérisé par le fait qu'il est constitué par un phage dont le ou les récepteurs bactériens correspondants sont distincts de celui auquel correspond la séquence de gène extracytoplasmique, qui est insérée dans ce vecteur.

10. Vecteur selon la revendication 9, caractérisé en ce que ce vecteur est dérivé du phage h°434.

11. Vecteur selon la revendication 10, caractérisé par le fait que la séquence insérée correspond au gène *lam*B de l'opéron maltose B.

12. Vecteur selon l'une quelconque des revendications 1 à 11, caractérisé par le fait que la séquence codant ladite protéine extracytoplasmique est formée d'un fragment à deux sites EcoRI comprenant le gène *lam*B, normalement contenu dans l'opéron maltose B.

13. Vecteur selon l'une quelconque des revendications 1 à 12, caractérisé par le fait qu'il comporte en outre un fragment comprenant lui-même le gène qui code une protéine étrangère déterminée, ce fragment étant inséré dans ledit vecteur en aval de ladite séquence d'insertion, mais en amont de l'extrémité, la plus éloignée du promoteur lactose, soit du gène Z ou fragment de gène Z, en aval de la susdite séquence, soit de la séquence de gène correspondant à la protéine extracytoplasmique, notamment *lam*B, dans le cas où cette séquence ne serait pas suivie d'un fragment distal de gène Z dans le sens de la traduction.

14. Application du vecteur selon la revendication 13 à la fabrication et l'excrétion de ladite protéine déterminée, comprenant notamment l'introduction dudit vecteur dans une bactérie-hôte, l'induction de l'expression de ce vecteur, notamment au moyen d'un inducteur de la β-galactosidase, et la récupération de la protéine hybride excrétée dans le milieu de culture.

15. Application du vecteur selon la revendication 13 à la production de vaccins à l'égard d'au moins une protéine antigénique, de préférence virale, caractérisée par le fait que la protéine déterminée susdite correspond à une protéine, notamment virale, à l'égard de laquelle une vaccination est recherchée, caractérisée par l'introduction dudit vecteur dans une cellule-hôte, notamment une bactérie-hôte, elle-même inoffensive, par l'induction de l'expression de ce vecteur, notamment au moyen d'un inducteur de la β-galactosidase, et par la récupération de la souche bactérienne porteuse de protéine hybride extracytoplasmique comprenant ladite protéine antigénique.

**Patentansprüche**

1. Vector, der den Promoter des Lactose-Operons und wenigstens ein Fragment des Z-Gens dieses gleichen Operons trägt, gekennzeichnet durch Einfügung von wenigstens einer Sequenz in diesen Vector, welche den proximalen Teil eines Gens umfasst, das wenigstens für einen Teil eines extracytoplasmischen, normalerweise in der Aussenmembran der ursprünglichen Wirtszelle befindlichen Proteins oder eines Proteins, welches fähig ist, in einem Kulturmedium dieser letztgenannten Zellen freigesetzt zu werden, codiert, wobei die Länge dieser Sequenz von extracytoplasmischem Gen ausreicht, um unter der Kontrolle des Promoters des Lactose-Operons die Ausscheidung eines Hybridproteins zu gestatten, welches Hybridprotein den besagten Teil des cytoplasmischen Proteins enthält und befähigt ist, in einer Zellkultur, insbesondere einer Kultur von einem Bakterienstamm, in deren Zellen dieser Vector vorher eingeführt worden ist, zur Expression gebracht zu werden.

2. Vector nach Anspruch 1, dadurch gekennzeichnet, dass die genannte eingefügte Sequenz aus jener Sequenz besteht, die befähigt ist, wenigstens für den proximalen Teil eines bakteriellen Empfängers, beispielsweise des Empfängers für den λ-Phagen, zu codieren.

3. Vector nach Anspruch 1 oder Anspruch 2, dadurch gekennzeichnet, dass die genannte eingefügte Sequenz in das Z-Gen oder in ein Fragment des Z-Gens oder unmittelbar unterhalb desselben eingefügt ist.

4. Vector nach einem der Ansprüche 1 bis 3, dadurch gekennzeichnet, dass die obgenannte Sequenz *lam*B-Gen sowie den distalen Teil des K-Gens umfasst, welch letztgenanntes in den Maltose B-Operons mit dem *lam*B-Gen assoziiert ist.

5. Vector nach Anspruch 4, dadurch gekennzeichnet, dass er die Sequenz S des *lam*B-Gens enthält, die den bakteriellen Empfänger für den λ-Phagen bestimmt, insbesondere ein proximales Fragment des *lam*B-Gens, welches seinerseits mit einem Fragment des Strukturgens der β-Galactosidase *lac*Z fusioniert ist.

6. Vector nach Anspruch 4 oder 5, dadurch gekennzeichnet, dass die Grösse des obgenannten proximalen Fragmentes des obgenannten *lam*B-Gens einem Proteinfragment mit einem Molekulargewicht, das wenigstens 2000, vorzugsweise 2500 erreicht, entspricht.

7. Vector nach Anspruch 6, dadurch gekennzeichnet, dass die Grösse des obgenannten proximalen Fragmentes des obgenannten *lam*B-Gens einem Proteinfragment mit einem Molekulargewicht, das wenigstens 20000, vorzugsweise wenigstens 30000 erreicht, entspricht.

8. Vector nach Anspruch 7, dadurch gekennzeichnet, dass die Grösse des obgenannten proximalen Fragmentes des obgenannten *lam*B-Gens einem Proteinfragment mit einem Molekulargewicht entspricht, welches zwischen ungefähr 20000 und ungefähr 40000, insbesondere zwischen ungefähr 35000 und ungefähr 40000, liegt.

9. Vector nach einem der Ansprüche 1 bis 8, dadurch gekennzeichnet, dass er aus einem Phagen besteht, dessen entsprechende(r) bakterielle(r) Empfänger verschieden von jenem bakteriellen Empfänger ist bzw. sind, welchem die in diesen Vector eingefügte Sequenz des extracytoplasmischen Gens entspricht.

10. Vector nach Anspruch 9, dadurch gekennzeichnet, dass dieser Vector von dem Phagen h°434 abgeleitet ist.

11. Vector nach Anspruch 10, dadurch gekennzeichnet, dass die eingefügte Sequenz *lam*B-Gen des Maltose-B-Operons entspricht.

12. Vektor nach einem der Ansprüche 1 bis 11, dadurch gekennzeichnet, dass die Sequenz, welche für das obgenannte, extracytoplasmische Protein codiert, aus einem Fragment gebildet ist, das zwei EcoRl-Stellen aufweist und das *lam*B-Gen umfasst, welches Fragment normalerweise im Maltose-B-Operon enthalten ist.

13. Vektor nach einem der Ansprüche 1 bis 12, dadurch gekennzeichnet, dass er ausserdem ein Fragment beinhaltet, welches seinerseits das Gen umfasst, welches für ein bestimmtes Fremdprotein codiert, wobei dieses Fragment in den genannten Vektor unterhalb von der genannten eingefügten Sequenz eingefügt ist, aber oberhalb zu dem in bezug auf den Lactose-Promoter am weitesten entfernten Ende entweder des Z-Gens oder des Fragmentes des Z-Gens unterhalb von der obgenannten Sequenz, oder der Gen Sequenz des dem extracytoplasmischen Protein entsprechenden Gens, insbesondere des *lam*B-Gens, entspricht, und zwar in jenem Fall, wo auf diese Sequenz kein distales Fragment des Z-Gens im Sinne der Translation folgen würde.

14. Anwendung des Vectors nach Anspruch 13 auf die Erzeugung und Ausscheidung des genannten bestimmten Proteins, umfassend insbesondere das Einführen des besagten Vectors in einen bakteriellen Wirt, die Induktion der Expression von diesem Vector, insbesondere mit Hilfe eines Induktors der β-Galactosidase, und die Gewinnung des in dem Kulturmedium ausgeschiedenen Hybridproteins.

15. Anwendung des Vectors nach Anspruch 13 auf die Erzeugung von Vakzinen in bezug auf wenigstens ein vorzugsweise virales Antigenprotein, wobei das genannte bestimmte Protein einem insbesondere viralen Protein entspricht, in bezug auf welches eine Impfung gesucht wird, gekennzeichnet durch das Einführen des besagten Vectors in eine Wirtszelle, insbesondere einen bakteriellen Wirt, der selbst unbedenklich ist, durch Induktion der Expression von diesem Vector, insbesondere mit Hilfe eines Induktors der β-Galactosidase, und durch die Gewinnung des Bakterienstammes, der das das besagte Antigenprotein umfassende, extracytoplasmische Hybridprotein trägt.

**Claims**

1. Vector carrying the promotor of the lactose operon and at least a fragment of the Z gene of the said operon, characterized by the insertion in said vector of at least one sequence comprising the proximal portion of a gene coding at least part of an extracytoplasmic protein normally located inside of the external membrane of the host-cell of origin or likely to be released in the culture medium of the latter, the length of this sequence of extracytoplasmic gene being sufficient to permit excretion, under the control of the promotor of the lactose operon, of a hybrid protein containing said part of cytoplasmic protein and liable of being expressed in a culture of cells, particularly of a bacterial strain, in which said vector will have been introduced beforehand.

2. Vector according to claim 1, characterized by the fact that said insertion sequence is constituted by that which is able to code for at least part of the proximal part of a bacterial receptor, such as the receptor for the λ phage.

3. Vector according to claim 1 or to claim 2, characterized in that said insertion sequence is inserted in the Z gene or Z gene fragment, or immediately downstream thereof.

4. Vector according to anyone of claims 1 to 3, characterized in that said sequence comprises the *lam*B gene and also the distal part of the K gene which, in the maltose B operon, is associated with gene *lam*B.

5. Vector according to claim 4, characterized by the fact that it contains the S sequence of the *lam*B gene determining the bacterial receptor for the λ phage, more particularly a proximal fragment of gene *lam*B and itself fused to a fragment of the β-galactosidase structural gene *lac*Z.

6. Vector according to claim 4 or 5, characterized by the fact that the size of said proximal fragment of said *lam*B corresponds to a proteic fragment having a molecular weight reaching at least 2,000, preferably 2,500.

7. Vector according to claim 6, characterized by the fact that the size of said proximal fragment of said *lam*B gene corresponds to a protein fragment having a molecular weight reaching at least 20,000, preferably at least 30,000.

8. Vector according to claim 7, characterized by the fact that the size of said proximal fragment of said *lam*B gene corresponds to a protein fragment having a molecular weight comprised between about 20,000 and about 40,000, particularly between about 35,000 and about 40,000.

9. Vector according to anyone of claims 1 to 8, characterized by the fact that it is constituted by a phage whose corresponding one or several bacterial receptors are distinct from that to which corresponds the sequence of extracytoplasmic gene which is inserted in said vector.

10. Vector according to claim 9, characterized in that this vector is derived of phage h°434.

11. Vector according to claim 10, characterized by the fact that the inserted sequence corresponds to the *lam*B gene of the maltose B operon.

12. Vector according to anyone of claims 1 to 11, characterized by the fact that the sequence coding for said extracytoplasmic protein is formed of a fragment with two EcoRl sites comprising the *lam*B gene, normally contained within the maltose B operon.

13. Vector according to anyone of claims 1 to 12, characterized by the fact that it comprises in addition a fragment comprising itself the gene which codes for a determined foreign protein, said fragment being inserted in said vector downstream of said insertion sequence, however upstream from the extremity which is the remotest from the lactose promoter, either of said Z gene or Z gene fragment, downstream of said sequence, or of the gene sequence corresponding to the extracytoplasmic protein, particularly *lam*B, in the case

13 to the production of vaccines with respect to at least one antigenic protein, preferably a viral one, characterized by the fact that said determined protein corresponds to a protein, particularly a viral one, with respect to which a vaccination is sought, characterized by the introduction of said vector in a host-cell, particularly a host-bacteria itself innocuous, by the induction of the expression of said vector, particularly by means of a β-galactosidase inductor, and by the recovery of the bacterial strain bearing the extracytoplasmic hybrid protein comprising said antigenic protein.

where said sequence would not be followed by a distal fragment of the Z gene in the direction of translation.

14. Application of the vector according to claim 13 to the production and excretion of said determined protein, comprising particularly the introduction of said vector into a host-bacterium, the induction of the expression of said vector, particularly by means of a β-galactosidase inductor, and the recovery of the hybrid protein excreted into the culture medium.

15. Application of the vector according to claim

# Fig.2.

$p\underline{lac}_{5\_1}$ —□▭— $\Delta Hin III$ ( ) $N\ CI_{857}$
Z

$f_1$

# Fig.1.

$\lambda\ ap\ \underline{mal}B12$ —▭— IBKEF $NCI_{857}$
$\underline{lam}B(EcoRI)$ (a)

$f_2$

## Fig.3.

$(I \simeq 146\%)$

—□▭ KEF $NCI_{857}$ —□▭— $\Delta Hin III$ ( ) $N\ CI_{857}$
Z' 'Z
b

$f_3$

## Fig.4.

$\lambda\ p\ \underline{mal}\ B\ 20$
$(I \simeq 90\%)$

—□▭ KEF $N\ CI_{857}$
Z'

$+\ \underline{lam}(EcoRI)$

de $\underline{mal}K^+$

## Fig.5.

$\lambda\ p\ \underline{mal}\ B\ 21$
$(I \simeq 100\%)$

—□▭ IBKEF $N\ CI_{857}$
Z'

Fig.6.

Fig.7.

Fig.8.

Fig.9.

OU

0 012 078

Fig.10a. $h\lambda$ $'AYZ''$ IBKEF $i21ts$ S7 $\lambda i^{21ts}$ S7 3221 (spi)

$z'-'b$

Fig.10b.

$\underline{I} \simeq 103$ % $\overset{\circ}{h}434$ $'AYZ'$ IBK' $'ZL$ $\Delta$Hin III CI857 $\lambda pY\overset{\circ}{h}434$ [IB]10

$I \simeq 102$ % $h\lambda$ $'AYZ'$ IBK' $'ZL$ $\Delta$Hin III CI857 $\lambda$LBZ

Fig.10c. $z'-'b$

Fig.10.

13

Fig.11.

$I \simeq 86\%$

$\overset{o}{h}\,434$    $\Delta$Hin III    $ZL$    att    $i^{21ts}$ nin$_5$    $\lambda$ plac$_{5-1}$ L8UV$_5$ i$^{21ts}$

nin$_5$ $\overset{o}{h}\,434$

$(=\lambda Z)$

[X] Eco RI

Fig.12a.

$I \simeq 86\% + Ix$

$\overset{o}{h}\,434$    $Z\downarrow$    $ZL$    att    $i^{21ts}$    $\lambda ZX$

x–z    nin$_5$

Fig.12b.

$I \simeq 102\%$

$h\lambda$    'A Y Z' 'IBK'    att    CI$_{857}$    $\lambda$LBZ

z'–'b    'ZB

Fig.12c.

$I \simeq 100,5\% + Ix$

$\overset{o}{h}\,434$    Z'X'Z''IBK'    att    CI$_{857}$    $\lambda$LBZ X$\overset{o}{h}\,434$

x–z'–'b    'ZL

Fig.12.

15